# EUROPEAN PATENT APPLICATION

(11) **EP 4 318 506 A2**
(43) Date of publication of application: **07.02.2024**
(21) Application number: 23218826.8
(22) Date of filing: 21.11.2019
(51) Int. Cl.: H01F 6/00

(54) **RAPID DUMP OF PARTIALLY INSULATED SUPERCONDUCTING MAGNET**

(30) Priority: 22.11.2018 GB 201819036; 11.04.2019 GB 201905168
(62) Divisional of application: 19809889.9
(71) Applicant: Tokamak Energy Ltd, Abingdon, Oxfordshire OX14 4SD (GB)
(72) Inventor: SLADE, Robert, Abingdon OX14 4SD (GB); VAN NUGTEREN, Bas, Abingdon OX14 5TN (GB)
(74) Representative: Marks & Clerk LLP

(57) **Abstract**

An HTS magnet system comprising an HTS coil and an insulating structure. The HTS coil comprising a plurality of turns comprising HTS material and metallic stabiliser; and an electrically conductive material connecting the turns. The insulating structure substantially surrounding the HTS coil, and having a breakdown voltage of less than 5kV and greater than 10V.

## Description

### Field of the Invention

The present invention relates to high temperature superconducting, HTS, magnets. In particular, the invention relates to a method for ramping down HTS magnets e.g. in response to quench detection, and apparatus implementing the method.

### Background

Superconducting materials are typically divided into "high temperature superconductors" (HTS) and "low temperature superconductors" (LTS). LTS materials, such as Nb and NbTi, are metals or metal alloys whose superconductivity can be described by BCS theory. All low temperature superconductors have a critical temperature (the temperature above which the material cannot be superconducting even in zero magnetic field) below about 30K. The behaviour of HTS material is not described by BCS theory, and such materials may have critical temperatures above about 30K (though it should be noted that it is the physical differences in composition and superconducting operation, rather than the critical temperature, which define HTS and LTS material). The most commonly used HTS are "cuprate superconductors" - ceramics based on cuprates (compounds containing a copper oxide group), such as BSCCO, or ReBCO (where Re is a rare earth element, commonly Y or Gd). Other HTS materials include iron pnictides (e.g. FeAs and FeSe) and magnesium diborate (MgB₂).

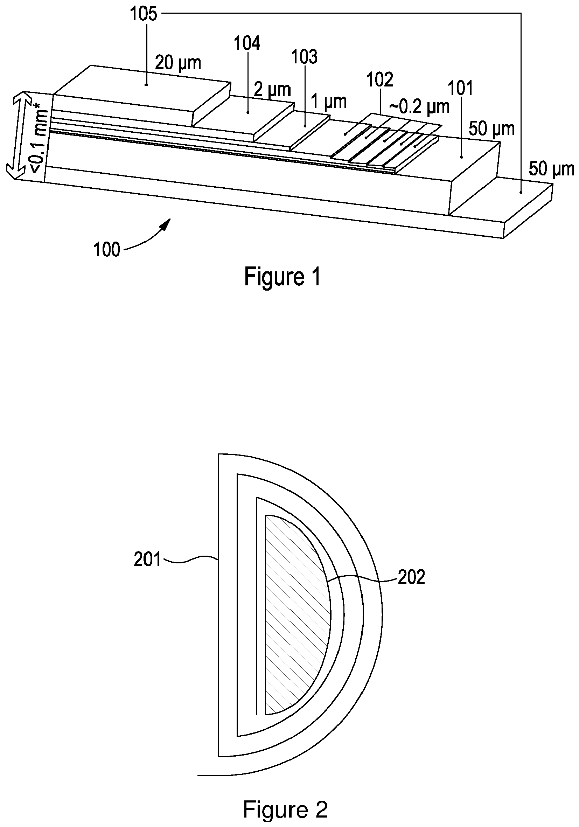

ReBCO is typically manufactured as tapes, with a structure as shown in Figure 1. Such tape 100 is generally approximately 100 microns thick, and includes a substrate 101 (typically electropolished hastelloy approximately 50 microns thick), on which is deposited by IBAD, magnetron sputtering, or another suitable technique a series of buffer layers known as the buffer stack 102,of approximate thickness 0.2 microns. An epitaxial ReBCO-HTS layer 103 (deposited by MOCVD or another suitable technique) overlays the buffer stack, and is typically 1 micron thick. A 1-2 micron silver layer 104 is deposited on the HTS layer by sputtering or another suitable technique, and a copper stabilizer layer 105 is deposited on the tape by electroplating or another suitable technique, which often completely encapsulates the tape.

The substrate 101 provides a mechanical backbone that can be fed through the manufacturing line and permit growth of subsequent layers. The buffer stack 102 is required to provide a biaxially textured crystalline template upon which to grow the HTS layer, and prevents chemical diffusion of elements from the substrate to the HTS which damage its superconducting properties. The silver layer 104 is required to provide a low resistance interface from the ReBCO to the stabiliser layer, and the stabiliser layer 105 provides an alternative current path in the event that any part of the ReBCO ceases superconducting (enters the "normal" state).

In addition, "exfoliated" HTS tape can be manufactured, which lacks a substrate and buffer stack, and instead has silver layers on both sides of the HTS layer. Tape which has a substrate will be referred to as "substrated" HTS tape.

HTS tapes may be arranged into HTS cables. An HTS cable comprises one or more HTS tapes, which are connected along their length via conductive material (normally copper). The HTS tapes may be stacked (i.e. arranged such that the HTS layers are parallel), or they may have some other arrangement of tapes, which may vary along the length of the cable. Notable special cases of HTS cables are single HTS tapes, and HTS pairs. HTS pairs comprise a pair of HTS tapes, arranged such that the HTS layers are parallel. Where substrated tape is used, HTS pairs may be type-0 (with the HTS layers facing each other), type-1 (with the HTS layer of one tape facing the substrate of the other), or type-2 (with the substrates facing each other). Cables comprising more than 2 tapes may arrange some or all of the tapes in HTS pairs. Stacked HTS tapes may comprise various arrangements of HTS pairs, most commonly either a stack of type-1 pairs or a stack of type-0 pairs and (or, equivalently, type-2 pairs). HTS cables may comprise a mix of substrated and exfoliated tape.

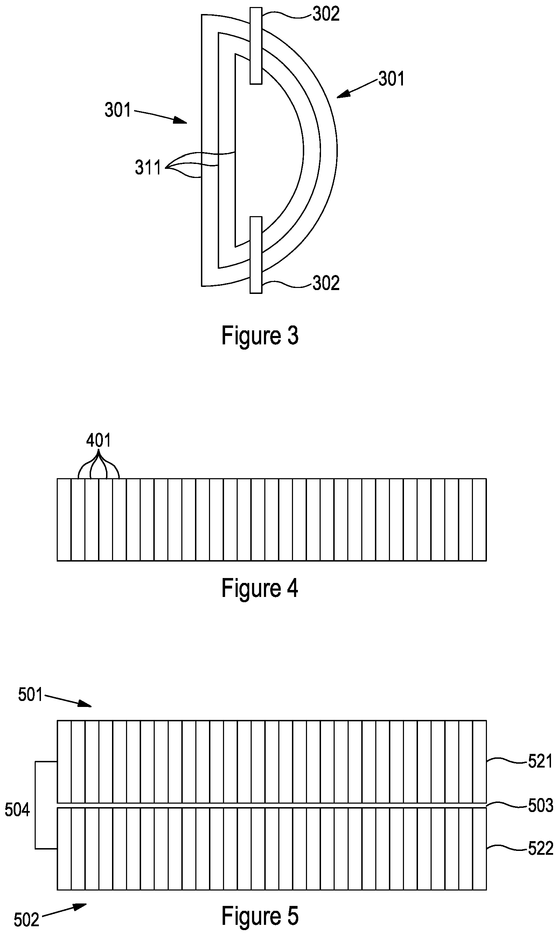

Broadly speaking, there are two types of construction for magnetic coils - by winding, or by assembling several sections. Wound coils, as shown in Figure 2, are manufactured by wrapping an HTS cable 201 around a former 202 in a continuous spiral. The former is shaped to provide the required inner perimeter of the coil, and may be a structural part of the final wound coil, or may be removed after winding. Sectional coils, as shown schematically in Figure 3, are composed of several sections 301, each of which may contain several cables or preformed busbars 311 and will form an arc of the overall coil. The sections are connected by joints 302 to form the complete coil. While the turns of the coils in figures 2 and 3 are shown spaced apart for clarity, there will generally be material connecting the turns of the coil - e.g. they may be consolidated by potting with epoxy.

Figure 4 shows a cross section of a specific type of wound coil known as a "pancake coil", where HTS cables 401 are wrapped to form a flat coil, in a similar manner to a spool of ribbon. Pancake coils may be made with an inner perimeter which is any 2 dimensional shape. Often, pancake coils are provided as a "double pancake coil", as shown in the cross section of Figure 5, which comprises two pancake coils 501, 502 wound in opposite sense, with insulation 503 between the pancake coils, and with the inner terminals connected together 504. This means that voltage only needs to be supplied to the outer terminals 521, 522, which are generally more accessible, to drive current through the turns of the coil and generate a magnetic field.

The coils may be "insulated" - having electrically insulating material between the turns of the coil, or "non insulated", where the turns of the coil are electrically connected radially, as well as along the cables (e.g. by connecting the copper stabiliser layers of the cables by soldering or by direct contact). "Partially insulated" coils are also possible, where the turn to turn resistance is less than that of a traditional insulator, such as ceramic or organic insulator, but greater than the resistance between tapes in a cable of the coil - e.g. between 100 and 10¹⁵ times the resistance between tapes in a cable. Having no or partial insulation between turns slows the rate at which the temperature of a local "hotspot" (normal zone) rises because it allows current to share between turns.

A non-insulated HTS coil can be modelled as having three current paths - two spiral paths, which follow the HTS tapes (one in the HTS and one in the metal stabilizer), and a radial path through the metal (and any other resistive material) connecting the non- or partially- insulated HTS cable turns between the coil terminals (while this can be modelled as a single path, it in fact represents the sum of all radial resistive paths through the magnet). Only current flowing in the spiral paths generates significant axial magnetic field in the centre of the coil. The HTS spiral path can be modelled as an inductor having a large inductance and zero or negligible resistance when the tape is all superconducting. The copper stabilizer spiral path is in parallel with the HTS path and has the same inductance, but significant resistance. For this reason negligible current flows in it unless parts of the HTS spiral path start to quench. If this happens the excess current above the critical current Ic of the HTS spiral path shares between the spiral stabilizer path and the radial path according to their relative resistances and L/R time constants. The radial current path can be modelled as having a negligible inductance and a much greater resistance than the spiral path while the HTS is superconducting throughout.

One potential use of HTS field coils is in tokamak plasma chambers, particularly for nuclear fusion reactors. WO 2013/030554 describes an approach involving the use of a compact spherical tokamak for use as a neutron source or energy source. The low aspect ratio plasma shape in a spherical tokamak improves the particle confinement time and allows net power generation in a much smaller machine. However, a small diameter central column is a necessity, which presents challenges for design of the plasma confinement magnet. High temperature superconductor (HTS) field coils are a promising technology for such magnets.

Another potential use of HTS field coils is in proton beam therapy devices. Proton beam therapy (PBT, also known as proton therapy) is a type of particle therapy used in the treatment of cancers (and other conditions which respond to radiotherapy). In PBT, a beam of protons is directed towards the treatment location (e.g. the tumour).

Another, similar therapy is proton boron capture therapy (PBCT), in which boron-11 is introduced to the target location, and a proton beam is used to initiate the p+¹¹B → 3α reaction. The same apparatus can be used to provide proton beams for either PBT or PBCT.

The proton beams for PBT and PBCT are generated by particle accelerators such as a cyclotrons or linear accelerators. Accelerators typically used for PBT and PBCT typically produce protons with energies in the range of 60 to 250MeV, with the most powerful currently operating facility having a maximum energy of 400 MeV.

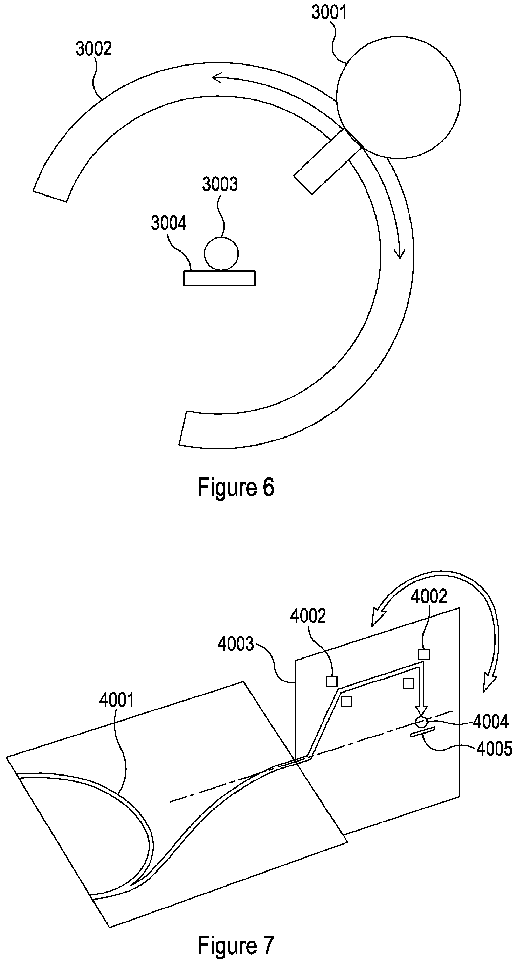

There are, broadly speaking, two types of design for PBT devices which allow variation of the beam angle. In the first type of design, as illustrated in Figure 6, the accelerator 3001 is mounted on a gantry 3002, which allows it to be rotated around the patient 3003 (usually about a horizontal axis). The patient is placed on a moveable bed 3004, which provides further degrees of freedom (e.g. translational motion and rotation about a vertical axis).

The second type of design is illustrated in Figure 7. The accelerator 4001 is stationary, and the beam is directed to the patient via steering magnets 4002 (generally including both quadrupole and dipole magnets), at least some of which are located on a gantry 4003, such that the beam can be rotated around the patient 4004 (e.g. about a horizontal axis). The patient is placed on a moveable bed 4005.

Either design requires that the gantry hold electromagnets capable of steering protons at the beam energy, which could be as high as 400MeV. This requires very high magnetic fields, and as such the use of HTS field coils can considerably reduce the mass and size of the electromagnets and the gantry needed to move them. HTS field coils may be used within the accelerator, quadrupole magnets of the steering magnets, or dipole magnets of the steering magnets.

### Summary

The invention is defined in the appended claims.

### Brief Description of the Drawings

Figure 1 is a schematic representation of an HTS tape;
Figure 2 is a schematic representation of a wound HTS coil;
Figure 3 is a schematic representation of a sectional HTS coil;
Figure 4 is a cross section of a pancake coil;
Figure 5 is a cross section of a double pancake coil;
Figure 6 is a schematic representation of a first PBT device;
Figure 7 is a schematic representation of a second PBT device;
Figure 8 shows results of a simulation of a partially insulated coil;
Figure 9 shows results of a simulation of an exemplary magnet system
Figure 10 shows graphs of magnetic field and temperature for a coil following simply shutting off the power supply;
Figure 11 shows the voltages for a coil during the same process as shown in Figure 10;
Figure 12A and B show an example of leaky insulation;
Figure 13A and B show a further example of leaky insulation;
Figure 14 shows an equivalent circuit to a two turn coil.

### Detailed Description

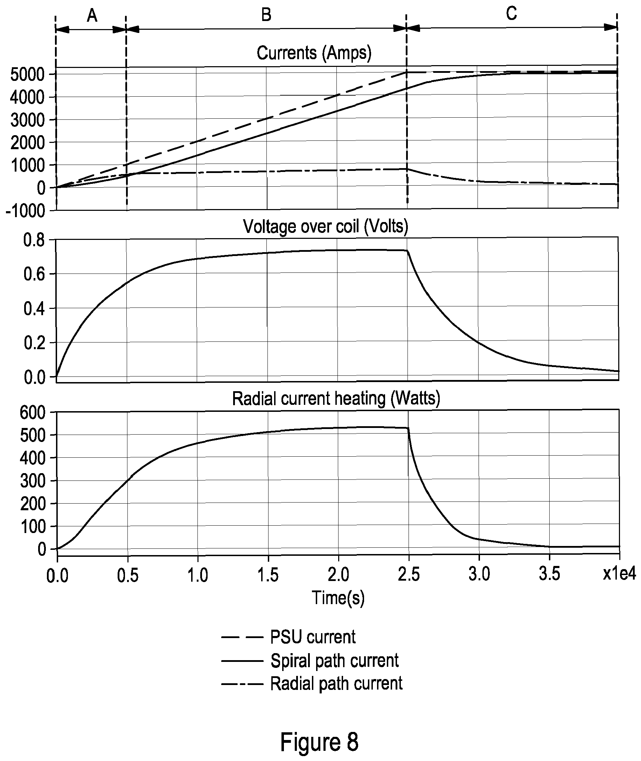

Figure 8 shows the current, voltage, and power in a non-insulated coil during ramp-up and into steady state operation. During ramp-up of a non-insulated coil, current will initially flow primarily in the radial path (period A in Fig 8), and then stabilize. The amount of current flowing in the radial path is higher for faster ramp rates (since the voltage developed across the spiral path, L.dl/dt, is higher - this is period B). At the end of the ramp, dl/dt drops to zero and current will transfer to the HTS spiral path with a time constant L/R (period C). The current will be mostly transferred into the spiral path a few (approximately five) L/R time constants after the end of the ramp. As such, the time constant should be selected to result in a reasonable ramp-up time, e.g. a time constant of 5-10 hours would be acceptable for the TF coil of a tokamak (giving a ramp-up time of about 1-2 days).

In a large magnet, in order to avoid damage from a quench (in either an insulated or non-insulated coil), an active quench protection scheme may also be implemented. In this scheme the magnet's stored energy may be dumped into some component other than the quenching region of the magnet before sufficient temperature rise can occur in the quenching region to cause damage. The other component may be an external resistance, or a separate portion of the magnet which is quenched over a larger proportion of the magnet's cold mass (thereby distributing the magnet's stored energy over a large volume, and reducing the overall temperature rise). However, the active approach requires the time between initiation of the normal zone (also called a "hot spot") and triggering of the magnet current ramp-down ("dump") to be short enough that the terminal temperature of the hot spot is less than a temperature at which damage to the coil can occur, eg: around 200 K. Such an approach may also be used in small magnets, to provide further protection against quenches.

The above, as well as discussion of suitable constructions to achieve a desired time constant, is discussed in more detail in co-pending application GB1818817.7

Although the use of PI coils extends the time available to dump the magnet current, it is still important that this operation is done as quickly as possible following detection of a hot spot. There has been relatively little discussion of current dumping techniques for PI coils, as in the literature PI coils are generally only used for smaller coils - in which the total energy of the coil is relatively low, and the quench will tend to propagate through the entire coil relatively quickly - meaning that the energy dump is spread through the coil. In addition, PI coils are inherently stable when compared to insulated coils, and so can often operate without quench protection as the risk is low. However, in large coils with considerable stored energy, coils with geometry that allows the hot spot to cover all the turns over only a relatively small proportion of the coil winding, and/or coils intended for long term operation in hostile environments (e.g. field coils for a nuclear fusion reactor), active quench protection is important.

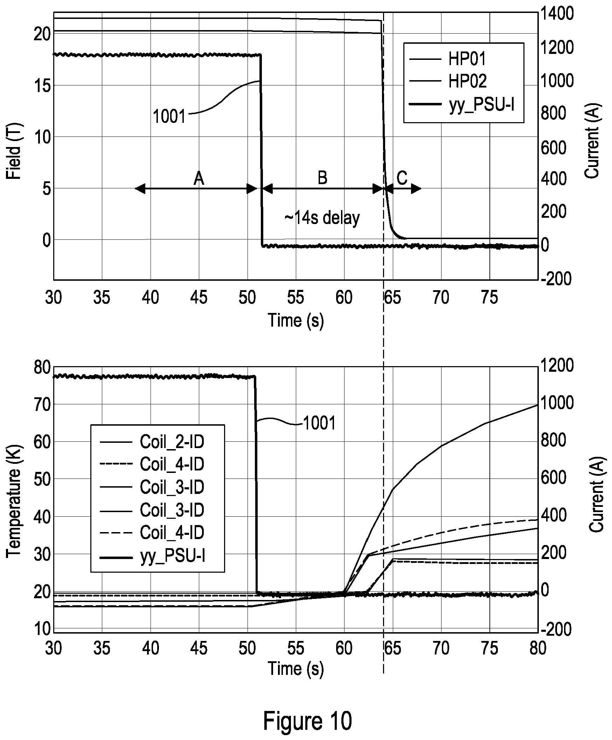

The use of PI coils provides further advantages when dumping energy from the magnet. Figure 10 shows graphs of magnetic field (proportional to spiral path current) and temperature for a coil following simply shutting off the power supply (i.e. making it an open circuit). During period A, the power supply is turned on (shown by the current 1001). At the start of period B, the power supply is shut off, causing the coil's inductance to generates a voltage which continues to drive the current in each turn in a closed loop, shorting back to the start of the turn via the turn-turn resistance. This causes ohmic heating which reduces the critical current in the HTS. This process continues for through period B (typically a few seconds, depending on many factors, 14 seconds in this experiment) until the HTS in the turn quenches and generates sufficient voltage to eject its loop current into the metal stabilizer in the spiral path (period C). Since this has a much higher resistance than the turn-turn resistance the turn's magnetic field energy is rapidly converted to heat in the stabilizer and the turn temperature rises uniformly as the spiral path current quickly drops to 0 (<1s).

However, for effective quench protection, a longer period B is still undesirable - in the event of a quench, this period may be long enough for significant local heating to occur in a local hot spot elsewhere in the magnet, resulting in an unacceptably high peak temperature in that hot spot. Furthermore, a magnet, such as a TF magnet in a tokamak, may comprise several coils (eg: separate limbs) that are poorly thermally and magnetically coupled, It is desirable that, when triggering a dump by turning off the power supply, all of the coils experience the same delay before quenching. Variations between coils may occur due to manufacturing differences of local temperature of magnetic field differences. If the coils quench non-simultaneously the resultant very large electromagnetic forces between coils may cause damage to the magnet's mechanical support structure, and/or the coils themselves. To minimise this it is desirable to minimise the variation between the delay between PSU off and quench in each coil, so that any variation between delays durations is also minimised. The length of this period could be reduced by increasing the resistance of the partially insulating layer (and thereby increasing the heating due to current travelling in the radial path in period B), but this would have knock-on effects to other electrical properties of the coil, e.g. altering the time constant for ramping up the coil, or making current sharing between turns more difficult (which will increase the likelihood of a hot-spot causing a global quench).

Instead, rather than shutting off the power supply, it is proposed to apply a large reverse current to the magnet coil (i.e. in the opposite direction to the current flowing through the coil prior to the ramp-down), e.g. by using a four-quadrant PSU which is able to sink current from the magnet coil. The superconducting path has a large inductance, and so this reverse current will flow primarily in the radial path in all the coils of the TF magnet. This large increase in radial current causes significant heating of all coils, rapidly quenching the entire magnet (and therefore spreading the energy dump over a large area).

While it may seem counterintuitive to apply a reverse current rather than simply shutting down the PSU, the important distinction is that the excess radial current will heat the whole bulk of the magnet - which means that the quench will quickly spread through the magnet and the energy dump will be spread through the whole volume (or at least a significant fraction). A concentrated energy dump, as would occur without any intervention, would cause unacceptable temperature rises in that small area, damaging the HTS. If a significant portion of the magnet is quenched, the same amount of energy (plus a small contribution from the reverse current itself) is spread throughout the magnet, which limits the temperature rise in the HTS. In addition, heating the magnet more evenly prevents steep temperature gradients from forming within the magnet. If the temperature gradient is too high, then the differing thermal expansion of nearby regions of the magnet will cause structural damage.

In existing magnets, even heating would be achieved by the use of "quench heaters" - i.e. heating elements laid adjacent to the HTS cable, which can be turned on to deliver heat to the coil. However, such heaters take up space, and as such reduce the available space for HTS conductor or for metal stabiliser. The "reverse current" method in effect uses the radial conduction path as a "quench heater", meaning that the heating is evenly distributed through the coil, and additional heating elements are not required.

The reverse current may be limited to, or set at, the operating current of the magnet. In this way, the maximum heating is achieved by the reverse current without exceeding the design parameters of components external to the magnet, compared to what would be required for normal magnet operation.

The above has been described with reference to quench protection, but it should be noted that the energy dumping technique described above may also be applicable to other situations where the magnet is ramped down - e.g. when shutting the magnet down under ordinary conditions, in the absence of any detected quench (or conditions likely to lead to a quench).

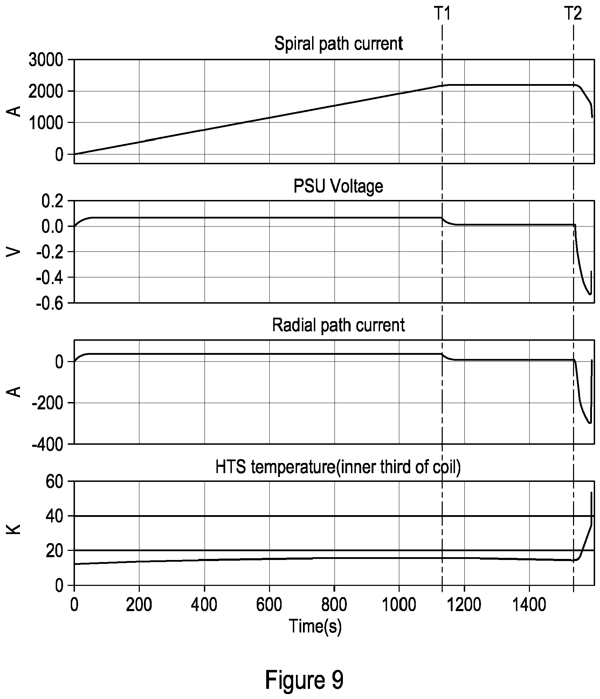

Figure 9 shows simulation results of an exemplary partially insulated coil during ramp-up, steady state operation, and ramp-down. In this case, the PSU (power supply) is modelled as a current source, i.e. the current supplied by the PSU is set in the simulation, and the voltage across the PSU is calculated.

During ramp-up the PSU current increases steadily to 2.2kA at a constant rate. The PSU voltage is positive, and on the order of 0.1V. The current in the radial path is approximately proportional to the PSU voltage (as the radial path can be modelled as a simple resistance), and the current in the spiral path increases at a constant rate. At time T1, when the desired current is reached, the magnet is switched to steady state operation - the PSU is set to a constant current, and the radial path current decreases with time constant L/R_{radial} as described previously. The PSU voltage decreases to a value on the order of a few millivolts when the radial path current has decayed, as the spiral path has negligible resistance (in this simulation, it is modelled as having zero resistance - so the PSU voltage tends to zero. In practice, it will generally settle around a few 10s-100s of millivolts). During ramp-up and steady state operation, the HTS temperature is substantially constant below 20K.

At time T2, a magnet dump is initiated (either in response to a quench detection or otherwise). The PSU supplies a reverse current (with a fast current ramp, modelled in this case as a downwards current ramp ten times faster than the initial ramp-up), which flows primarily in the radial path. The PSU voltage is negative during the supply of this current, on the order of -0.5V. The HTS temperature rises quickly. The simulation ends when the HTS temperature reached approx 55K, as the entire coil will quench, and the temperature rise becomes too fast for the model used. However, in reality the magnet's stored energy would be rapidly converted to heat, spread relatively uniformly over the magnet, safely shutting it down.

The reverse current may be supplied for a set time, or until a specified condition is reached - e.g. on detection of a quench in a substantial portion of the magnet, detection of a specified temperature in a substantial portion of the magnet, or detection that the spiral path current (or the magnetic field generated by the coil) has decreased below a threshold value.

The speed of the ramp-down is dependent on the speed of the reverse current ramp in the PSU .

It is desirable to be able to control the rate of change of current during both the magnet ramping phase and the dump phase. For this reason, a PSU with feedback controlled current output is preferred. The PSU current may be controlled on the basis of the current in the spiral path, the temperature of the magnet, the magnetic field generated by the coil, or any other suitable property of the coil.

The power supply may comprise multiple power supply units, each of which provides power to the coil during a different period. In particular, the power supply may comprise a first unit for supplying power to the coil during ramp-up and steady state operation, and a second unit for supplying the reverse voltage to the coil during ramp-down.

The power supply (or one or more power supply units of the power supply) may be partially located within the cryostat containing the HTS magnet, and may comprise a transformer arranged to transfer power across the cryostat without having cables passing through the cryostat, as described in PCT/GB2018/050337.

Where ramp-down of the magnet is triggered in response to detection of a quench or conditions likely to lead to a quench, this detection may be by any practical method. For example:
- detection of an excess voltage across the HTS material in the magnet;
- the use of secondary HTS tapes which are provided adjacent to the main coil, and configured to quench before the main coil, e.g. as described in international patent application PCT/GB2016/052712 or UK patent application GB1812120.2
- detection of temperature, strain, magnetic fields, or other conditions within the magnet coil, e.g via Raleigh scattering in fibre optic cables as described in international patent application PCT/GB2017/053066 or via other temperature, strain, or magnetic field detectors as known in the art.

It is important not to apply reverse current for too long, otherwise the active dump could warm the magnet above 200K and cause problems. Ideally the dump system should limit the total energy applied to the magnet to that required to raise the temperature of the whole magnet above it's critical temperature (ie: turn all coils from superconducting to normal). This is a small fraction of the total energy needed to raise the whole magnet to -200K. As the coils begin to quench, the magnet's own stored energy will be dissipated, driving the global quench.

A simple way to apply the correct amount of energy is to discharge a capacitor bank into the magnet. This also avoids the need for a four-quadrant PSU. A single quadrant PSU may be used to ramp the magnet. When it is necessary to dump the magnet this is simply disconnected using active switches and a pre-charged capacitor bank connected across the magnet to drive the reverse radial current. Note that no large voltages are generated by disconnecting the PSU since the large inductance of the magnet is shunted by its radial resistance

Most of the above disclosure has been focussed on ramping down a magnet following quench detection, where the speed of the ramp-down is of critical importance. There are also techniques using the same underlying principle which are applicable to controlling the magnet in conditions where the ramp-down time is not the primary factor..

As an example, the PSU may be configured to supply a ramp-down current which is less than the current in the magnet, but in the same direction. This will cause a current equal to the difference between the magnet and PSU currents to run in the radial path, heating the magnet as before. This will result in a slower ramp down compared to a reverse current (or simply disconnecting the PSU), and a reduced temperature rise in the magnet.

As a further example, the PSU may be configured to supply an AC current overlaid on the DC delivered to the coil (either during steady state, ramp-up, or ramp-down). Where the period of the AC current is significantly less than the time constant L/R, this AC current will flow entirely in the radial path. This results in heating of the magnet without otherwise affecting the current in the spiral path (compared to the case where only the DC current is delivered).

The overlaid AC current may also be used in combination with any of the previous examples. For example, a combination of a DC reverse current and an AC current (i.e. where the total current is a sinusoidal current with an average value which is opposite in sign to the coil current, and a period less than the time constant of the magnet) may be used to ramp down the coil with additional heating. Alternatively, the AC current may be combined with a DC ramp down current which is less than the current in the magnet, but in the same direction (i.e. where the total current is a sinusoidal current with an average value which is less than the coil current, and a period less than the time constant of the magnet). As a further alternative, a purely AC current may be provided to ramp down the magnet - this will cause a current equal to the magnet current to run in the radial path, in addition to the AC current. In each case, adding the AC current results in a greater current in the spiral path (and hence quicker ramp down) compared to using only the DC current.

A surprising feature of ramping down a partially insulated coil as discussed in the above examples (either by shutting down the PSU or by providing a modified current) is that the turn to turn voltage of the coil remains low (on the order of a few volts, even for large coils) throughout the process. Large insulated superconducting coils require heavy duty insulation, which can withstand very high voltages - but the small voltages experienced by a partially insulating coil could be insulated against effectively by a much wider variety of materials (or even a simple vacuum or air gap).

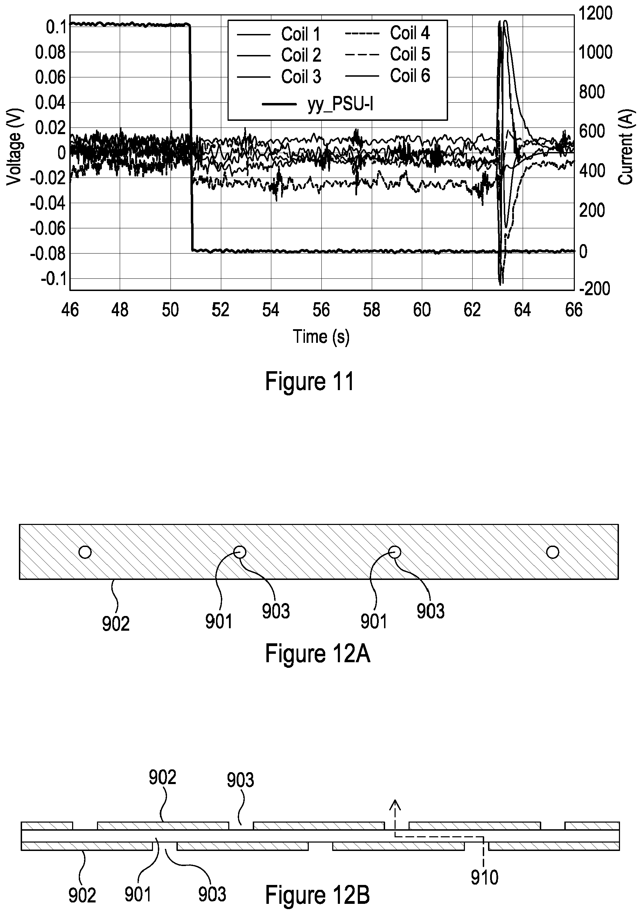

Figure 11 shows the voltage across each coil in the same magnet as used in Figure 10 (which contains 6 pancake coils, with a total inductance of 0,12H). The peak voltages, which occur at a time corresponding to the start of the global quench (i.e. the end of period B and beginning of period C in figure 10) are about 0.1V. In contrast, the expected voltage for an equivalent insulated coil under the same conditions would be on the order of at 2kV, and the expected voltage for a large coil would be at least 5kV (the voltage can be calculated as V=-L dl/dt, where dl/dt is approximately the transport current (1.4kA for the test coil) divided by the time taken to quench (0.1 kA for the test coil)). In a partially insulated coil, the coil can be operated at high transport current and inductance without a particularly high voltage developing - which is most practical when operating a large coil at low transport currents (a few kiloamps, compared to typically 50kA) and high inductance (i.e. a higher number of turns).

The peak voltage generated between two turns of a partially insulating coil during current dump with an open circuit PSU can be approximated as V=I₀R_{stab}, where I₀ is the transport current, and R_{stab} is the resistance of the resistive material in the spiral path of the coil.. The quench propagates between coils by mutual inductance, which means that the peak coil voltage does not exceed the peak voltage for a single turn. The peak voltage will not exceed 10V for any realistically large coil.

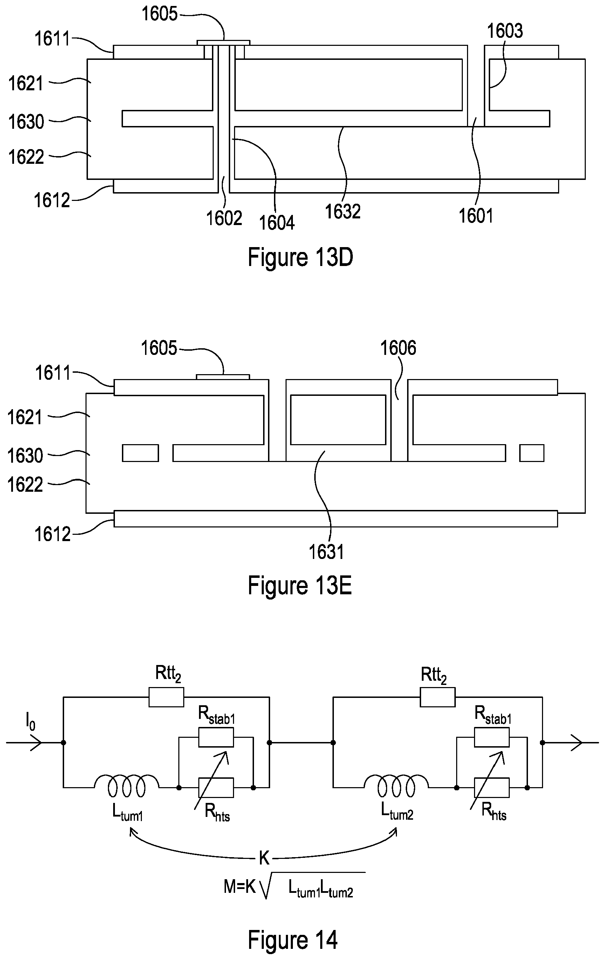

Figure 14 shows the equivalent circuit for a coil with 2 turns (and can be extended to more turns by adding more coils in series, and mutual inductances between each coil). In this figure, I₀ is the transport current, Rₜₜ is the turn to turn resistance (i.e. the radial path resistance divided by the number of turns), R_{stab} is the resistance of the resistive spiral path, Lₜᵤᵣₙ is the induction of each turn, and R_{HTS} is the resistance of the HTS in each turn (i.e. 0 during normal operation, and only non-zero during a quench or near-quench). M is the mutual induction between the two turns, and K is the coupling coefficient between the turns.

This is of particular importance for insulation of the coil - both for insulation of the coil as a whole from other components ("ground wrap"), and for designs of partial insulation which use insulating material having a number of conductive channels through it (known as "leaky insulation", and discussed in more detail below). The low voltage means that the insulation does not need to be a heavy duty material such as Kapton^{™} (as used in conventional insulated magnets), but simpler materials such as paint, varnish, or even paper can be used, or the insulating material can be replaced by an air or vacuum gap (with suitable support structures, also insulators if required).

Insulating structures can be characterised by a "breakdown voltage", above which the structure ceases insulating and the resistance of the structure drops from the order of several megaOhms to the order of a few Ohms or milliOhms. For an insulated coil, this breakdown voltage would need to be at least 2kV (at least 5kV for large coils, e.g. with a radius greater than 50cm), which severely limits the materials that can be used while still keeping the insulation reasonably compact (the breakdown voltage is approximately proportional to the thickness of the material, with the proportionality constant ("dielectric constant") depending on the material).

For a partially insulating coil, only a breakdown voltage greater than 10V or so is required - which would allow the use of any material that can be reasonably called an "insulator"

Alternatively, in environments hostile to materials (e.g. in a fusion reactor, where the materials will be subject to neutron bombardment), this allows for insulation to be used for longer before degrading to the point where it is no longer insulating - as neutron bombardment will tend to cause changes in insulation which will reduce its dielectric constant, or introduce physical gaps.

An example of leaky insulation is shown in Figure 12A and B. The metal strip 901 is provided with a thin insulating coating 902 on at least the sides facing the HTS cables, where the insulating coating is removed or missing over windows (or "through holes") 903 at intervals on each side of the metal strip. The windows can have any shape and can extend to the edges of the tape. The location of windows on either side of the metal strip are staggered, as shown in Figure 12B, which increases the resistance (compared to an uninsulated strip, or to a strip where the windows on each side were directly opposite each other) as the current must take a path 910 along part of the length of the metal strip.

By varying the spacing of the windows such that they are closer together in the return limbs and further apart in the core, the required difference in turn to turn resistance between the return limbs and core can be achieved. Further tuning may be achieved by using a different metal for the metal strip in the core compared to in the return limbs, or by varying other aspects of the geometry of the strip.

To allow for even further tuning, rather than a solid metal strip, a layer having several metal tracks may be used - effectively forming an insulating layer having conductive radial tracks disposed within it, where the spacing and length of the tracks determines the resistance of the partially insulating layer.

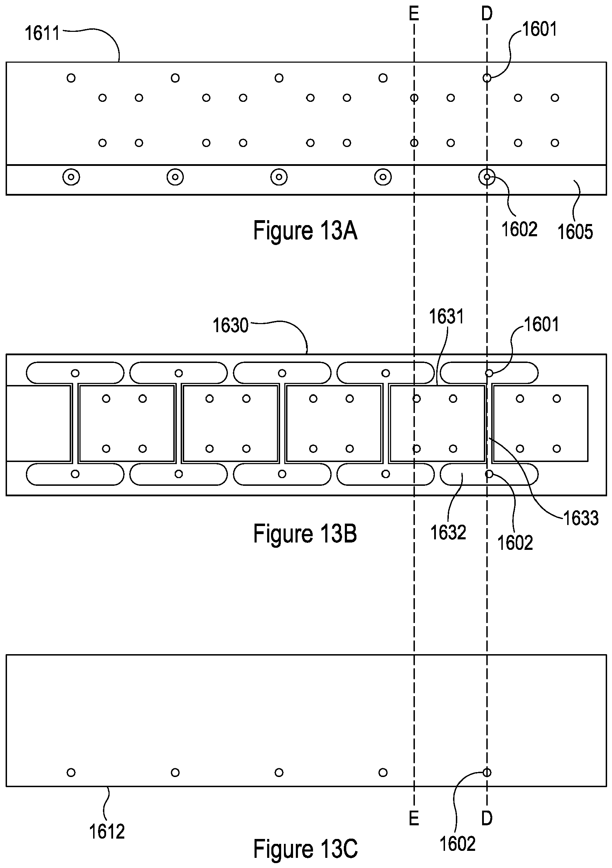

Figures 13A to E show a further example leaky insulation layer. The leaky insulation layer comprises 5 layers, which are, in order:
- a first metal connection layer 1611;
- a first insulating layer 1621;
- an electrically conducting layer 1630;
- a second insulating layer 1622;
- a second metal connection layer 1612.

Figures 13A to C show the layout of the first metal connection layer 1611, electrically conducting layer 1630, and second metal connection layer 1622 respectively. Figures 13D and E are cross sections along the lines D and E in Figures 13A to C.

The connection layer is present to facilitate attachment to HTS cables by soldering.

In contrast to the previous example where the electrically conducting layer is a continuous metal strip, in this example the electrically conducting layer is divided into several conductive regions. These regions come in two types. The square regions 1631 (though they may be any shape in practice) are connected by vias 1606 only to one of the metal connection layers. These regions do not affect the electrical properties of the partially insulating layer, but provide a thermal path through the respective insulating layer. By varying the size of these regions and the number of connections between them and the metal connection layer, the thermal properties of the partially insulating layer can be varied independently of the electrical properties.

The other regions 1632 each connect a window 1601 of the first insulating layer 1621 to a window 1602 of the second insulating layer 1622. The resistance between the windows can be controlled by varying the geometry of the regions 1632 - e.g. where the region 1632 contains a track 1633 which is elongate as shown in Figure 13B, increasing the width of the track would reduce the resistance between the windows, and increasing the length of the track (e.g. by providing a non-linear track, or by moving the windows) would increase the resistance between windows.

The windows 1601 in the first insulating layer are formed by drilled vias through the first connection layer and the first insulating layer, which are then plated with metal 1603 (or other electrically conductive material) to connect the first connection layer and the electrically conductive layer. The windows 1602 in the second insulating layer are formed by drilling a via 1602 through all of the layers, which is then plated with metal 1604 (or other electrically conductive material). To prevent a connection being formed to the first connection layer through the windows 1602 of the second insulating layer, the first connection layer is etched around the via 1602 to electrically isolate it, and an insulating cap 1605 is placed on the end of the via 1602 to ensure no bridging occurs due to soldering or contact with the HTS cable.

As an alternative, the windows 1602 may instead be drilled from the other side of the partially insulating layer, such that they pass through the second connection layer, second insulating layer, and electrically conducting layer, and do not pass through (or do not pass completely through) the first insulating layer. As a further alternative, all the windows may be formed from vias which pass through all layers, with etching of the second connection layer and an insulating cap on the second connection layer being used for windows 1601 of the first insulating layer.

Another unexpected advantage of partially insulated coils is that the additional quench stability allows a greater choice of materials for the non-superconducting conductive elements within each cable. In conventional coils, both the stabiliser of the HTS (i.e. the thin layer of metal or metal cladding on each tape) and any material connecting the tapes would be copper - as it has a very low resistivity, and higher resistivity materials would cause excessive heating. However, copper is also a relatively soft metal, so under high pressures it can be squeezed out of the tape, or can deform under shear stresses which is likely to be responsible for the damage to the HTS layers following a quench.

Therefore, it is preferable to reduce or eliminate copper from the turns of the coil and the partial insulation. Reduced copper may be, for example, less than 10 microns thickness of copper per HTS tape in the coil (i.e. reduced compared to conventional HTS tapes), or less than 5 microns thickness of copper (i.e. less than half that). The metals or other electrical conductors used in place of copper may have one or more of:
- reduced ductility compared to copper;
- an increased shear modulus compared to copper;
- an increased Young's modulus compared to copper;
- an increased bulk modulus compared to copper;
- an increased Brinell Hardness Number compared to copper.

Suitable materials include stainless steel.

The above disclosure can be applied to a variety of HTS magnet systems. In addition to the tokamak fusion reactor mentioned above as an example, it may be used for HTS coils in nuclear magnetic resonance imaging (NMR / MRI) devices, manipulation of magnetic devices within a non-magnetic medium via magnetic fields (e.g. robotic magnetic navigation systems for manipulating medical devices within a patient), and magnets for electric motors, e.g. for electronic aircraft. As a further example, the disclosure may be applied to proton beam therapy devices comprising HTS magnet systems which include the disclosed features, where the HTS magnet systems are used within the accelerator of the PBT device, the quadrupole or dipole steering magnets of the PBT device, or any other magnet of the PBT device.

## Claims

1. A high temperature superconducting, HTS, magnet comprising:
a high temperature superconducting, HTS, coil comprising:
a plurality of turns comprising HTS material and metallic stabiliser;
an electrically conductive material the turns providing an electrical connection between the turns;
an insulating structure substantially surrounding the HTS coil, and having a breakdown voltage of less than 5kV and greater than 10V.

2. An HTS magnet according to claim 1, wherein the insulating structure is a coat of a paint or varnish.

3. An HTS magnet according to claim 1 or 2, wherein the insulating structure comprises a gap filled with gas or vacuum, and a support structure configured to structurally connect the HTS coil to other components of the magnet.

4. An HTS magnet according to any of claims 1 to 3, wherein the insulating structure has a breakdown voltage of less than 2kV, less than 1 kV, less than 500V, or less than 100V.

5. An HTS magnet according to any of claims 1 to 4, wherein the electrically conductive material comprises an electrically conductive layer separating the turns.

6. A high temperature superconducting, HTS, coil, comprising:
a plurality of turns comprising HTS material and metallic stabiliser;
a partially insulating layer separating the turns, wherein the partially insulating layer comprises:
an electrically conductive material, the electrically conductive material providing an electrical path between adjacent turns, and
an insulating structure restricting the electrical paths through the partially insulating layer;
wherein the insulating structure has a breakdown voltage of less than 5kV and greater than 10V.

7. An HTS coil according to claim 6, wherein the insulating structure comprises a gap filled with gas or vacuum.

8. An HTS coil according to claim 6 or 7, wherein the insulating structure does not comprise any material having a dielectric constant greater than 5kV volts divided by the minimum separation between adjacent turns.

9. An HTS coil according to any of claims 6 to 8, wherein the electrically conductive material is formed as:
an electrically conductive layer having the insulating structure on each side; and
a plurality of electrically conductive links extending through the insulating structure to connect the electrically conductive layer to the respective turns.

10. An HTS coil according to claim 9, wherein the electrically conductive layer is divided into regions separated by portions of the insulating structure, each region connecting an electrically conductive link connected to a first coil to an electrically conductive link connected to a second coil.

11. A high temperature superconducting, HTS, coil, comprising:
a plurality of turns comprising HTS material and metallic stabiliser;
a partially insulating layer separating the turns, wherein the partially insulating layer comprises:
an electrically conductive material providing an electrical path between adjacent turns;
a gap filled with gas or vacuum, the gap being arranged to restrict the electrical paths through the partially insulating layer.

12. A high temperature superconducting, HTS, coil, comprising:
a plurality of turns comprising HTS material and metallic stabiliser;
a partially insulating layer electrically connecting the turns;
wherein the turns do not contain copper, or the turns contain copper with a total thickness less than 10 microns of copper per HTS conductor in the turn.

13. An HTS coil according to claim 12, wherein the partially insulating layer does not contain copper.

14. An HTS coil according to claim 12 or 13, wherein the turns and/or partially insulating layer comprise an electrical conductor having one or more of:
reduced ductility compared to copper;
an increased shear modulus compared to copper;
an increased Young's modulus compared to copper;
an increased bulk modulus compared to copper;
an increased Brinell Hardness Number compared to copper.

15. An HTS coil according to claim 14, wherein the turns and/or partially insulating layer comprise steel.
